# EUROPEAN PATENT SPECIFICATION

(11) **EP 0 481 701 B1**
(45) Date of publication and mention of the grant of the patent: **04.10.1995**
(21) Application number: 91309420.7
(22) Date of filing: 14.10.1991
(51) Int. Cl.: A61K 9/50, A61K 9/52, A61K 9/127, A61K 47/48, A61K 47/42, A61K 39/395, A61K 7/16

(54) **Treatment composition**
Behandlungsmittel
Composition de traitement

(30) Priority: 15.10.1990 GB 9022298
(43) Date of publication of application: 22.04.1992
(73) Proprietor: UNILEVER PLC, London EC4P 4BQ (GB); UNILEVER N.V., 3013 AL Rotterdam (NL)
(72) Inventor: Francis, Sheila Elizabeth, Calow, Chesterfield, Derbyshire (GB); Jones, Malcolm Norcliffe, Sale, Cheshire M33 2EU (GB); Hutchinson, Fiona Jane, Hale, Altrincham, Cheshire WA15 8BX (GB); Lyle, Ian Gardner, Aston Park, Deeside, Clywd CH5 1XQ (GB)
(74) Representative: Ford, Michael Frederick

(56) References cited:
- EP-A- 0 036 277
- EP-A- 0 224 837
- WO-A-86/04232
- WO-A-90/07924
- WO-A-90/10448
- US-A- 3 911 099
- US-A- 4 470 967
- US-A- 4 762 915
- US-A- 4 767 615

## Description

This invention relates to compositions to be used for the delivery of oral benefit agents to target sites in the mouth. In particular the invention is applicable to the delivery of therapeutic agents active against organisms which give rise to dental plaque.

The so-called "magic bullet" concept refers to a therapeutic agent which is somehow bound to some entity having an affinity for the intended target site. It is envisaged that with such an arrangement the therapeutic agent will be carried to its intended target and will not display significant activity at other possible destinations which it may have an opportunity of reaching. A particular application of this which is frequently envisaged is the delivery of an anti cancer drug which is also somewhat toxic. If such a drug is formulated as a "magic bullet" it will, so it is hoped, be delivered to the cancer against which it is desired to act and not to other parts of the body where the drug would merely produce undesirable side effects.

The "magic bullet" is frequently envisaged as comprising a microcapsule of some description as a carrier for the therapeutic agent, with surface structures providing a means of molecular targeting. Possible carriers include polymeric nanocapsules and liposomes. Liposomes are small sacs formed from certain surface active molecules, most commonly phospholipids, which in aqueous media arrange themselves into a bi-layered membrane defining a microscopic closed vesicle.

It has been proposed to use liposomes to constitute "magic bullet" type drugs in which the therapeutic agent is enclosed within the liposome which carries the drug to the target site while also keeping the therapeutic agent out of contact with alternative targets which are bypassed on the way to the target site.

US4767615 discloses delivering therapeutic agents in liposomes which have affinity for hydroxyapatite (an inorganic material). This might give selectivity as to where in the mouth receives the therapeutic agent. However, this approach could not be expected to improve delivery more generally, since the amount of exposed hydroxyapatite is, in practice, likely to be small.

WO-A-90/07924 relates to a method for coupling a targeting molecule to a liposome. The liposome may be used to encapsulate, and to target delivery of, various benefit agents including diagnostic agents, fungicides, insect repellents and the like. No reference is made to the delivery of oral benefit agents.

EP-A-0,452,268 was published after the priority date of the present application, and is relevant to the novelty only of the present claims, for the purposes of designated states Belgium, Germany, Denmark, Spain, France, the United Kingdom, Greece and Italy (Article 54(3) EPC). The document relates to the use of microcapsules as carriers for an active agent (in particular a buffer, for use as an anti-caries agent) to achieve sustained release of that agent, especially in the oral cavity. The microcapsules are made from natural gums, so that they adhere to surfaces in the mouth.

We have now found that liposomes with means to attach to specific organic surfaces can be used to give unexpectedly effective delivery of therapeutic agents to target sites in the mouth following topical application.

Such effective delivery is unexpected because the liposome encapsulating the therapeutic agent has a much larger bulk than the same agent when molecularly dispersed, so that the mobility of the former in solution would be lower, and diffusion would lead to slower delivery to the target site. Also, if a therapeutic agent is incorporated into a product to be used in the mouth, such as for instance a dentifrice then the mouth would be the first part of the human body to be exposed to this product. There are no alternative targets for the therapeutic agent to bypass on the way.

Surprisingly we have found that when the duration of exposure to the composition is short (which of course is a realistic situation for a target site in the mouth) a therapeutic agent is rendered more effective when it is enclosed in liposomes which have means for binding it to the desired target site.

Accordingly, the present invention provides a composition for topical application and containing microcapsules, particularly liposomes, which enclose an oral benefit agent active at a target location in the mouth and which have on their surfaces molecular structures capable of recognising and binding specifically to components of dental plaque.

The composition will generally include a vehicle suitable for topical application in the mouth.

This invention also relates to a method of delivering a non-therapeutic oral benefit agent to a target location in the mouth, by incorporating the agent in microcapsules as above which are in turn incorporated into a composition for topical application. The invention further comprises use of the microcapsules in preparing such compositions.

A preferred composition contains liposomes which enclose a therapeutic agent, active against oral bacteria, and which have on their surface molecular structures capable of recognising and binding to particular components of dental plaque.

The liposomes may be prepared from those materials which are known for the purpose; examples are given in J H Fendler, "Membrane Mimetic Chemistry" (Wiley-Interscience, New York, 1982) and in J N Weinstein and J D Leserman, Pharmac, Ther., 1984 24, 207-233. Among the materials most commonly used are phospholipids from natural sources such as lecithin from egg or soya, and synthetic analogues such as L-α-dipalmitoyl phosphatidylcholine (DPPC). Charged phospholipids such as phosphatidyl inositol are often incorporated in liposomes to improve colloidal stability.

Techniques for preparation of liposomes are described in G Gregoriadis, "Liposome Technology - Vol 1", (CRC Press, 1984) and in P R Cullis et al., "Liposomes - from Biophysics to Therapeutics", Chapter 5, (Ed. M J Ostro, Marcel Dekker, New York, 1987). The methods which we prefer are sonication (in an ultrasonic bath) of a phospholipid dispersion and reverse phase evaporation. Another method which may be used is "extrusion" under pressure through very fine passages such as provided by Nuclepore (RTM) membranes.

The techniques lead to somewhat different liposomes. Sonication and extrusion generally lead to small vesicles, less than 100 nm in diameter. Reverse phase evaporation leads to larger vesicles having diameters ranging from 100 nm up to several microns.

Liposomes may act as carriers for water-soluble, oil-soluble or microcrystalline solid materials. Incorporation of water-soluble material into liposomes is normally accomplished by including the material into the aqueous solution during liposome formation. When the liposomes are formed, some of the solution becomes enclosed within the vesicles. Oil-soluble materials may be incorporated by including the material in the lipid mixture prior to formation of the liposomes; such materials are normally sequestered in the lipid membrane. Microcrystalline solid materials may be incorporated by internal precipitation of insoluble salts when one of the constituent ions is encapsulated and the other is allowed to permeate through the liposomal membrane from the external solution.

The organic material for which the microcapsules have affinity may be for example a protein, glycoprotein or carbohydrate exposed on the surface of the oral cavity. Possible targets include the proteins of tooth pellicle, polysaccharides of mucosal surfaces such as gums, tongue and cheeks, and extracellular polysaccharides of oral bacterial species such as Streptococci, Actinomyces, Lactobacilli and Bacteroides (which form dental plaque). Particularly preferred targets are Streptococcus mutans or Streptococcus sanguis.

The molecular structures used to bind the microcapsules to the components of dental plaque are at the exterior of the microcapsules and is a molecule having strong affinity for said components, for example a specifically binding protein, polysaccharide, glycoprotein, lipoprotein or lipopolysaccharide. One such type of molecule is an antibody, which might be monoclonal or polyclonal. Monoclonal antibodies may be generated using the technique first described by Kohler and Milstein (Nature, 256, 495-479, 1975). Antibodies may, alternatively be generated by the use of recombinant DNA techniques, for example as described in US Patents 4816397 and 4816567. Specific binding subunits or antibody fragments may also be used. These may be generated by enzymic digestion of intact antibody molecules, for example using papain or pepsin, or may be produced using the recombinant DNA techniques described in the two US patents referred to above.

A further possibility is to use a lectin bound to the outer surface of the microcapsules. Lectins are plant-derived proteins with binding affinity for certain sugar groups which occur in polysaccharides and glycoproteins. Their binding affinity tends to be less specific than that of antibodies.

Both antibodies and lectins can be bound to liposomes by covalent bonds. Various techniques are known for conjugating proteins to other materials and generally entail providing each of the two species with one of a pair of materials which will react together. Suitable methods are described in M J Ostro, "Liposomes - from Biophysics to Therapeutics", Chapter 5 (Marcel Dekker, New York, 1987) and in F J Martin et al., "Liposomes - A Practical Approach", Chapter 4 (Ed R R C New, IRL Press, Oxford, 1990). Our preferred technique for attaching to liposomes is to react m-maleimidobenzoyl-N-hydroxysuccinimide (MBS) with a component of the phospholipid mixture before formation of the liposomes and to react N-succinimidyl-S-acetylthioacetate (SATA) with the protein. After liposome formation the residues of these substances are coupled together in aqueous medium under mild conditions.

The use of these materials to conjugate antibodies and lectins to liposomes has been described in F.J. Hutchinson et al., Biochim. Biophys. Acta., 978 (1989) 17-24.

The oral benefit agent which is incorporated within the microcapsules may be for example a therapeutic agent, in particular an anti-bacterial agent effective against bacteria which cause dental plaque, an anti-inflammatory agent capable of reducing gingivitis, an anti-tartar agent, an anti-caries agent, or a tooth desensitising agent. Alternatively, a non-therapeutic agent may be incorporated, for example a flavour for sustained breath refreshment.

Of particular interest are antibacterial agents having molecular weight not greater than 2000. Within this category biphenolic compounds are of interest. A preferred benefit agent is Triclosan, (2,4,4'-trichloro-2'-hydroxy diphenyl ether) which is a broad spectrum antibacterial agent active against common oral pathogens.

Preferred possibilities for forms of composition containing the microcapsules are a mouthwash and a gel. Other possibilities include a toothpaste and a lozenge able to dissolve in the mouth.

Proportions may vary widely. However, the benefit agent will generally provide from 0.01 to 50% by weight of the microcapsules which in turn will generally provide from 0.01 to 50% by weight of the composition. The benefit agent will generally provide from 0.001 to 10% by weight of the composition.

The benefit agent preferably provides from 0.1 to 30% more especially 0.5 to 10% by weight of the microcapsules. The microcapsules will preferably constitute 0.1 to 10%, more especially 1 to 5% by weight of the composition. The benefit agent preferably provides from 0.01 to 5%, better 0.05 to 1% by weight of the overall composition.

### Example 1

Liposomes were prepared by the reverse phase evaporation procedure referred to above incorporating Triclosan in the lipid mixture. Some of the liposome samples were prepared with concanavalin A (ConA) conjugated to their surface. Details are as follows:
(a) Derivatisation of ConA with SATA 2.5µl of a stock solution containing 9.08mg N-succinimidyl-S-acetyl thioacetate (SATA) in 50µl dimethylformamide was added to ConA solution (10mg in 2.5ml phosphate (50mM) - EDTA (1mM) buffer, pH7.5) at room temperature. After reaction (15min) the derivatised protein (s-ConA) was separated from unreacted SATA by gel filtration on a Sephadex G-50 column (15 x 2cm). The s-ConA was activated by deacetylation using, for each 2ml of protein solution, 200µl of 0.1M hydroxylamine solution, made up in 2.5mM EDTA with sufficient solid Na₂HPO₄ added to bring the pH to 7.5.
(b) Preparatlon of DPPE-MBS
40mg L-α-dipalmitoylphosphatidylethanolamine (DPPE) was dissolved in a mixture of 16ml dry chloroform, 2ml dry methanol and 20mg dry triethylamine. 20mg maleimidobenzoyl-N-hydroxysuccinimide ester (MBS) was added and the reaction mixture was stirred under nitrogen at room temperature for 24h, after which the organic phase was washed three times with phosphate buffered saline (PBS, pH7.3) to remove unreacted MBS. The DPPE-MBS derivative was recovered from the organic phase by rotary evaporation and was stored in a chloroform/methanol mixture (9:1 v/v) at 4°C.
(c) Preparation of liposomes
27mg dipalmitoylphosphatidylcholine (DPPC), 3mg phosphatidylinositol (PI, from wheat germ) and 3mg DPPE-MBS were dissolved in 9ml chloroform/methanol mixture (4:1 v/v). 4mg Triclosan was added to this solution when encapsulation of the antibacterial agent was required; [¹⁴C]-DPPC and [³H]-Triclosan were incorporated as radiotracers in some experiments. A 3ml aliquot of this solution was placed in a 50ml round bottomed flask and the solvent removed by rotary evaporation (60°C) to yield a thin lipid film. The film was redispersed in 6ml 4:1 chloroform/methanol, 3ml of nitrogen-saturated 1:10 diluted PBS was added at 60°C and the mixture gently shaken, followed by 3min sonication under nitrogen at 50°C. The resulting homogenous emulsion was rotary evaporated at 60°C until, after passing through an intermediate viscous 'gel' phase, it inverted to a water-continuous system. The aqueous liposomal dispersion was then purged with nitrogen for a further 15min at 50°C to remove traces of organic solvent and was kept at that temperature for another 15min to allow annealing to occur.
Liposome dispersions thus prepared were passed through a Sephadex G-50 gel filtration column (30x2cm) pre-equilibrated with PBS. The fractions collected were analysed for phospholipid and Triclosan by scintillation counting. Particle sizes of the liposomes were determined by photon correlation spectroscopy, following the method described in Hutchinson et al., Biochim. Biophys, Acta, 978 (1989) 17-24.
(d) Conjugation of ConA to liposomes
Some samples were prepared with concanavalin A covalently coupled to the surface of the liposomes. This was accomplished simply by mixing and equilibrating aliquots of the dispersion with the deacetylated SATA derivative of ConA in appropriate proportions at room temperature for 2 hours or at 4°C overnight. After conjugation the reaction mixture was applied to a Sepharose 4B column to separate the proteoliposomes from unreacted protein.
(e) Assessment of efficacy
The antibacterial efficacy of Triclosan delivered in targeted liposomes was assessed using a bacterial regrowth assay. The oral bacterium Strep. sanguis (CR2b) was grown for 18h at 37°C in medium containing 10% BHI, 0.3% yeast extract and 1% sucrose. The bacteria were harvested by centrifugation (2000rpm, 5min), washed 3 times with sterile PBS and resuspended in PBS to give an optical density at 550nm of 0.5. 100µl of suspension (containing 7.2 x 10⁵ cells by viable cell counting) was pipetted into a microtitre plate well and left overnight to adsorb. After incubation the well was washed twice with 300µl sterile PBS, and the plate was blotted dry; the number of cells remaining was 1.4 x 10⁵. Non-specific binding sites were then blocked by treatment for 30min at 20°C with a sterile solution of 0.02% w/v casein in PBS, after which the well was washed 3 times with PBS and the plate blotted dry.
100µl of test solution containing either 10µg free Triclosan or liposomes with or without 10µg Triclosan, all in PBS containing 10% ethanol (Triclosan is virtually insoluble in PBS alone; this level of ethanol does not kill the bacteria), was added to the well and allowed to adsorb for 2h at 37°C. The plate was then washed 3 times with sterile PBS, 100µl growth medium added (10% BHI plus 0.3% yeast extract and 1% sucrose), sealed and incubated statically in a candle jar for 18h at 37°C. After this incubation period, the plate was read using a Dynatech MR610 plate reader and the extent of continuing bacterial growth determined from the measured optical density at 630nm.
(f) Results

| Treatment solution | % inhibition of growth* |
|---|---|
| Triclosan in solution | 75 |
| Triclosan in liposomes without ConA | 12 |
| Triclosan in liposomes with ConA | 38 |

| | |
|---|---|
| * relative to PBS alone | |

Liposomes without ConA were 1.096 x 10⁻⁶ moles lipid per ml, including 23.97% Triclosan by weight, and had weight average diameter 251nm.

Liposomes with ConA were 9.61 x 10⁻⁷ moles lipid per ml, including 13.67% Triclosan by weight, had weight average diameter 213nm and weight average number of 814 ConA molecules per liposome.

Free Triclosan was 10µg in 100µl of PBS containing 10% ethanol. Total Triclosan in liposome systems was 10µg for liposomes with ConA and 20µg for liposomes without ConA.

It can be seen that with the long (2 hour) contact time, delivery in liposomes reduced the effectiveness of the Triclosan. Liposomes conjugated to concanavalin A were better than liposomes without this lectin at their surface, but not as effective as Triclosan alone.

### Example 2

The procedures of Example 1 were repeated using similar liposomes, but with a slightly lower level of Triclosan and with the time of exposure to the treatment solutions reduced from 2h to 1min, to more realistically simulate use of a product intended to give topical application in the mouth.

This was carried out using PBS as in Example 1 for some samples, and PBS plus human saliva (100µl of 1:1 mixture of saliva with sterile PBS) for others to give a closer representation of oral conditions. Comparative experiments again used Triclosan in solution in PBS containing 10% ethanol.

| Results | % inhibition of growth | |
|---|---|---|
| Treatment solution | incubated with saliva | without saliva |
| Triclosan in solution | 3 | 8 |
| Triclosan in liposomes with concanavalin A | 26 | 20 |

Liposomes had weight average diameter 339nm and weight average number of 1467 molecules of concanavalin A per liposome.

All treatment systems contained 5µg Triclosan in 100µl PBS with 10% ethanol.

It can be seen that when the period of exposure to the treatment solution is short (a realistic situation), the liposomes greatly increase the antibacterial action compared with Triclosan in free solution.

## Claims

1. A composition for topical application and containing microcapsules which enclose an oral benefit agent active at a target location, accessible by topical application, in the mouth, the microcapsules having on their surfaces molecular structures capable of recognising and binding specifically to components of dental plaque.

2. A composition according to claim 1, wherein the microcapsules are liposomes.

3. A composition according to claim 1 or claim 2, wherein the molecular structures comprise an antibody or antibodies or fragment thereof,

4. A composition according to claim 1 or claim 2, wherein the molecular structures comprise a lectin or lectins.

5. A composition according to claim 3 or claim 4, having the antibody(ies) or lectin(s) covalently conjugated to the microcapsule.

6. A composition according to any one of the preceding claims, wherein the oral benefit agent is an anti-bacterial agent.

7. A composition according to claim 6, wherein the anti-bacterial agent is a biphenolic compound having a molecular weight of less than 2000.

8. A composition according to any one of the preceding claims which is a mouthwash, a toothpaste or a lozenge able to dissolve in the mouth.

9. A method of delivering a non-therapeutic oral benefit agent to a target location in the mouth, the method comprising incorporating the benefit agent in microcapsules which have on their surfaces molecular structures capable of recognising and binding specifically to components of dental plaque; and applying topically in the mouth a composition containing the said microcapsules.

10. The use, in the preparation of a composition for topical application in the mouth, of microcapsules which enclose an oral benefit agent active at a target location, accessible by topical application, in the mouth, the microcapsules having on their surfaces molecular structures capable of recognising and binding specifically to components of dental plaque.

11. A method of manufacturing a composition including an oral benefit agent active at a target location, accessible by topical application, in the mouth, the method comprising enclosing the benefit agent in microcapsules and providing said microcapsules on their surfaces with molecular structures capable of recognising and binding specifically to components of dental plaque.

## Patentansprüche

1. Mittel zur örtlichen Anwendung und enthaltend Mikrokapseln, die ein durch örtliche Anwendung zugängliches Mundpflegemittel, das an einem Zielort im Mund wirksam wird, einschließen, wobei die Mikrokapseln auf ihren Oberflächen Molekülstrukturen aufweisen, die in der Lage sind, spezifisch Komponenten von Zahnplaque zu erkennen und daran zu binden.

2. Mittel nach Anspruch 1, wobei die Mikrokapseln Liposome sind.

3. Mittel nach Anspruch 1 oder Anspruch 2, wobei die Molekülstrukturen einen Antikörper oder Antikörper oder Fragmente davon umfassen.

4. Mittel nach Anspruch 1 oder Anspruch 2, wobei die Molekülstrukturen ein Lectin oder Lectine umfassen.

5. Mittel nach Anspruch 3 oder Anspruch 4 mit kovalent an die Mikrokapsel konjugiertem(n) Antikörper(n) oder Lectin(en).

6. Mittel nach einem der vorangehenden Ansprüche, wobei das Mundpflegemittel ein antibakterielles Mittel ist.

7. Mittel nach Anspruch 6, wobei das antibakterielle Mittel eine biphenolische Verbindung mit einem Molekulargewicht von weniger als 2000 ist.

8. Mittel nach einem der vorangehenden Ansprüche, nämlich ein Mundwasser, eine Zahncreme oder eine Pastille, die in der Lage ist, sich im Mund aufzulösen.

9. Verfahren zur Abgabe eines nichttherapeutischen Mundpflegemittels an einen Zielort im Mund, wobei das Verfahren umfaßt, Einschluß des Pflegemittels in Mikrokapseln, die auf ihrer Oberfläche Molekülstrukturen aufweisen, welche in der Lage sind, spezifisch Komponenten von Zahnplaque zu erkennen und daran zu binden und örtliche Anwendung eines diese Mikrokapseln enthaltenden Mittels im Mund.

10. Verwendung von Mikrokapseln, welche ein durch örtliche Anwendung zugängliches, an einem Zielort im Mund wirksames Mundpflegemittel einschließen, wobei die Mikrokapseln auf ihren Oberflächen Molekülstrukturen aufweisen, die in der Lage sind, spezifisch Komponenten von Zahnplaque zu erkennen und daran zu binden, bei der Herstellung eines Mittels zur örtlichen Anwendung im Mund.

11. Verfahren zur Herstellung eines Mittels, das ein durch örtliche Anwendung zugängliches, an einem Zielort im Mund wirksames Mundpflegemittel einschließt, wobei das Verfahren Einschluß des Pflegemittels in Mikrokapseln und Versehen der Mikrokapseln an ihren Oberflächen mit Molekülstrukturen, die in der Lage sind, Komponenten von Zahnplaque spezifisch zu erkennen und daran zu binden, umfaßt.

## Revendications

1. Composition pour application locale et contenant des microcapsules qui enferment un agent bénéfique oral actif au niveau d'un emplacement cible, accessible par une application locale, dans la bouche, les microcapsules ayant à leurs surfaces des structures moléculaires capables de reconnaître des composants de la plaque dentaire et de se lier spécifiquement à ceux-ci.

2. Composition selon la revendication 1, dans laquelle les microscapsules sont des liposomes.

3. Composition selon les revendications 1 ou 2, dans laquelle les structures moléculaires comportent un anticorps ou des anticorps ou un fragment de ceux-ci.

4. Composition selon la revendication 1 ou la revendication 2, dans laquelle les structures moléculaires comportent une lectine ou des lectines.

5. Composition selon la revendication 3 ou la revendication 4, ayant l'anticorps ou les anticorps ou la lectine ou les lectines conjugués de manière covalente à la microcapsule.

6. Composition selon l'une quelconque des revendications précédentes, dans laquelle l'agent bénéfique oral est un agent anti-bactérien.

7. Composition selon la revendication 6, dans laquelle l'agent anti-bactérien est un composé biphénolique ayant un poids moléculaire inférieur à 2000.

8. Composition selon l'une quelconque des revendications précédentes qui est un collutoire, un dentifrice ou une pastille capable de se dissoudre dans la bouche.

9. Procédé d'administration d'un agent bénéfique oral non-thérapeutique au niveau d'un emplacement cible de la bouche, le procédé comportant l'incorporation de l'agent bénéfique dans des microcapsules qui ont à leurs surfaces des structures moléculaires capables de reconnaître des composants de la plaque dentaire et de se lier spécifiquement à ceux-ci; et l'application de manière locale au niveau de la bouche d'une composition contenant lesdites microcapsules.

10. Utilisation, dans la préparation d'une composition destinée à une application locale au niveau de la bouche, de microcapsules qui enferment un agent bénéfique oral actif au niveau d'un emplacement cible, accessible par application locale, dans la bouche, les microcapsules ayant à leurs surfaces des structures moléculaires capables de reconnaître des composants de la plaque dentaire et de se lier spécifiquement à ceux-ci.

11. Procédé de fabrication d'une composition incluant un agent bénéfique oral actif au niveau d'un emplacement cible, accessible par application locale, dans la bouche, le procédé consistant à enfermer l'agent bénéfique dans des microcapsules et à munir lesdites microcapsules sur leurs surfaces de structures moléculaires capables de reconnaître des composants de la plaque dentaire et de se lier spécifiquement à ceux-ci.
